# EUROPÄISCHE PATENTANMELDUNG

(11) **EP 1 083 452 A1**
(43) Veröffentlichungstag der Anmeldung: **14.03.2001**
(21) Anmeldenummer: 00119264.0
(22) Anmeldetag: 06.09.2000
(51) Int. Cl.: G02B 21/08

(54) **Beleuchtungseinrichtung für ein Operationsmikroskop**

(30) Priorität: 09.09.1999 CH 165199
(71) Anmelder: Leica Microsystems AG, 9435 Heerbrugg (CH)
(72) Erfinder: Pensel, Jürgen, 9450 Altstätten (CH); Sander, Ulrich, 9445 Rebstein (CH)

(57) **Zusammenfassung**

Die Erfindung betrifft eine Beleuchtungseinrichtung mit einer Blende (8) für ein Operationsmikroskop, deren Blende (8) einen Teillichtstrom der Beleuchtung abzudecken vermag, wobei die Abdeckung wegen der besonderen Blendenausbildung nicht vollständig erfolgt.

## Beschreibung

Die Erfindung betrifft eine Beleuchtungseinrichtung für ein Operationsmikroskop, bei der der gesamte Lichtstrahl aus einer, einer Lampe zugeordneten Beleuchtungsoptik durch ein erstes Prisma aus einer Senkrechten auf die Hauptachse des Hauptobjektives des Mikroskops in eine Schräge mit kleinem Winkel zur Hauptachse umgelenkt wird, und bei der ein zweiter Teil der gesamten Lichtstrahlenmenge etwa parallel zur Hauptachse auf das Operationsfeld geworfen wird. Dieser Aufbau wird insbesondere für Augenoperationen verwendet. Im Zentrum des Operationsfeldes befindet sich die Beleuchtung annähernd parallel zur Operationsmikroskopachse, während sie rund um dieses Zentrum unter kleinem Winkel in Richtung Operationsfeld strahlt. Während der Operation wird in der Regel der Beleuchtungsanteil unter kleinem Winkel durch eine einschiebbare Blende ausgeblendet, sodass der Operateur lediglich der parallelen Beleuchtungsanteil zur Verfügung hat. Dadurch ist allerdings auch das tatsächlich beleuchtete Operationsfeld auf den Durchmesser der Beleuchtung mit parallelem Anteil eingeengt.

Dies wurde in der Praxis fallweise als Nachteil empfunden, sodass der Erfindung die Aufgabe zugrunde liegt, eine verbesserte Abblendwirkung zu erzielen, die jedoch etwas Licht auch für den Bereich unter kleinem Winkel, sodass dieser Bereich nicht vollständig abgedunkelt wird.

Ein naheliegender Lösungsschritt ist, die bereits standardmässig vorgesehene Blende nicht vollständig vor den Prismenausgang zu setzen, sondern einen kleinen Spalt für geringen Lichtdurchtritt freizulassen, was bei einigen Geräten von den Anwendern auch so gemacht wird. Versuche ergaben allerdings, dass diese Lösung insofern nicht befriedigend ist, als sich starke Verzeichnungen, Vignettierungen und über das Beleuchtungsfeld verteilte Beleuchtungsstellen mit uneinheitlichen Helligkeitswerten ergaben.

Dieses Problem wird durch die Erfindung dadurch verhindert, dass neu anstelle einer lichtundurchlässigen Blende eine Blende mit geringer Lichtdurchlässigkeit eingesetzt wird. Gelöst wird diese Aufgabe erstmals durch den Aufbau gemäss Anspruch 1.

Eine solche Blende kann ein neutrales Farbfilter, eine Lochblende oder ein Blendengitter o. dgl. sein. Gemäss einer Weiterentwicklung dieser Erfindung könnten die lichtdurchlässigen Stellen der Blenden auch mit optischen Elementen versehen werden, sodass es zu einer Streuung des Lichtes kommt, die nach Bedarf das Operationsfeld sogar über die bisher standardmässig beleuchtete Fläche hinaus, beleuchtet. Anstelle solcher optischer Elemente oder zusätzlich dazu könnten auch Markierungen, wie z.B. Fadenkreuze o.dgl. in das Leuchtfeld abgebildet werden.

Dem Anwender ist somit auch - allerdings nur schwach - neben dem Operationsfeld eine gute Wahrnehmung z.B. von seitlich eingebrachten OP-Instrumenten und/oder eine zusätzliche Signalisierung über die Markierungen möglich.

### Stand der Technik:

Die DE-C2- 40 28 605 und ihr Familienmitglied US-A- 5,126,877 stellen eine bekannte Beleuchtungseinrichtung vor. Eine noch ältere, an sich gut funktionierende Beleuchtungseinrichtung wurde durch die Vorgängerin der Anmelderin unter der Bezeichnung "Beleuchtungsoptik 0°" bereits um 1985 auf den Markt gebracht (vgl. Prospekt M1 668d-X.85 aus Oktober 1985 der Wild Heerbrugg AG). Durch Drehen einer Scheibe kann bei der "Beleuchtungsoptik 0°" wahlweise zu einer Standardbeleuchtung in einem kleinen Winkel zur Hauptachse eine "0°-Beleuchtung" hinzugefügt werden, die im gleichen Ausmass ihrer Lichtstromzunahme den Lichtstrom der Standardbeleuchtung reduziert. Theoretisch ist dabei eine Steuerung der Lichtstromverteilung möglich.

So bewährt dieser bekannte Aufbau ist, sosehr wurde nach neueren Lösungen gesucht, die an sich keine Lichtstromumverteilung vornehmen; d.h. von der einen Lichtstromart keine Lichtstrommengensubstraktion zugunsten der anderen Lichstromart vornehmen. Eine solche Lösung findet sich in den Eingangs erwähnten DE-C und US-A.

Ein noch wesentlich jüngerer, bekannter Aufbau ist in der JP-A-9-105866 angegeben. Etwa vergleichbar mit dem alten Aufbau "0°-Beleuchtung" der Vorgängerin der Anmelderin findet sich dort unter einem ersten Prisma ein zweites Prisma mit zwei Spiegelflächen, das allerdings nicht um die Hauptachse drehbar ist. Dieser Aufbau führt zu einer grundsätzlich konstanten Lichtstromaufteilung zwischen dem 0°-Lichtstromteil und jenem grösseren Lichtstromanteil, der permanent unter kleinem Winkel einfällt, wobei eine gemeinsame Reduktion des Lichtstromes durch eine einschiebbare Blende im Hauptlichtstrahlengang der Beleuchtungsoptik möglich ist.

### Weitere Varianten und Weiterebildungen der Erfindung:

Eine einfache Lösung mit gleichmässiger Ausleuchtung des betroffenen Objektbereiches ergibt sich an der besonderen Ausbildung gemäss Anspruch 2.

Die Anordnung der Blende vor den Lichteintriffsflächen ist ebenso möglich, wie die Anordnung der Blende vor den Lichtaustrittsflächen der Licht- Teil und Umlenkvorrichtung.

Die Erfindung ist jedoch auf diese angegebenen Blenden nicht eingeschränkt, sondern kann vielmehr mit jeder bekannten oder neuen Lichtreguliervorrichtung ausgerüstet sein, ohne ihr Wesen, nämlich die teilabdunkelnde Bauart des Lichstromes unter kleinem Winkel zu verlieren.

Eine besondere Ausgestaltung und gegebenenfalls eine Alternative zu dem bisher Beschriebenen, die alternativ oder auch in Kombination sinnvoll angewendet werden kann und bei Bedarf zudem zu einer verbesserten noch symmetrischeren Beleuchtung führt, ist in Anspruch 8 angegeben. Durch diesen Aufbau kann beim Abdunkeln des einen Bereiches zusätzlich Licht für den anderen zur Verfügung gestellt werden - bei unveränderter Leistung der Lichtquelle.

Unabhängig oder zusätzlich zur Lichtstromregelung kann erfindungsgemäss auch eine Lichtfarbenregelung vorgesehen sein, wie in Anspruch 10 angegeben.

Anhand der Zeichnung wird die Erfindung beispielhaft näher erläutert.
Es zeigen dabei die
Fig. 1 die Draufsicht auf wesentliche Elemente einer Variante der Erfindung;
Fig.2 die erweiterte Seitansicht eine anderen Variante mit schematischer Beleuchtungsoptik und mit einem Hauptobjektiv ;
Fig.3 ein Beispiel einer erfindungsgemässen Blendenscheibe,
Fig. 4 eine Variante mit Spiegeln anstelle von Prismen, bei denen die neuartigen erfindungsgemässen Blenden alternativ oder gleichzeitig vor beiden Eintrittsflächen der Licht-Teil- und Lenkvorrichtung vorschiebbar sind und
Fig.5 eine Variante, die der alten 0° Beleuchtung der Anmelderin am ähnlichsten ist, jedoch ebenso mit einer neuen Blende ausgerüstet wurde.

Die Figuren werden zusammenhängend beschrieben. Gleiche Bezugszeichen bedeuten gleiche Bauteile. Gleiche Bezugszeichen mit unterschiedlichen Indizes bedeuten Teile mit gleichen Aufgaben bzw. ähnlichen Funktionen.

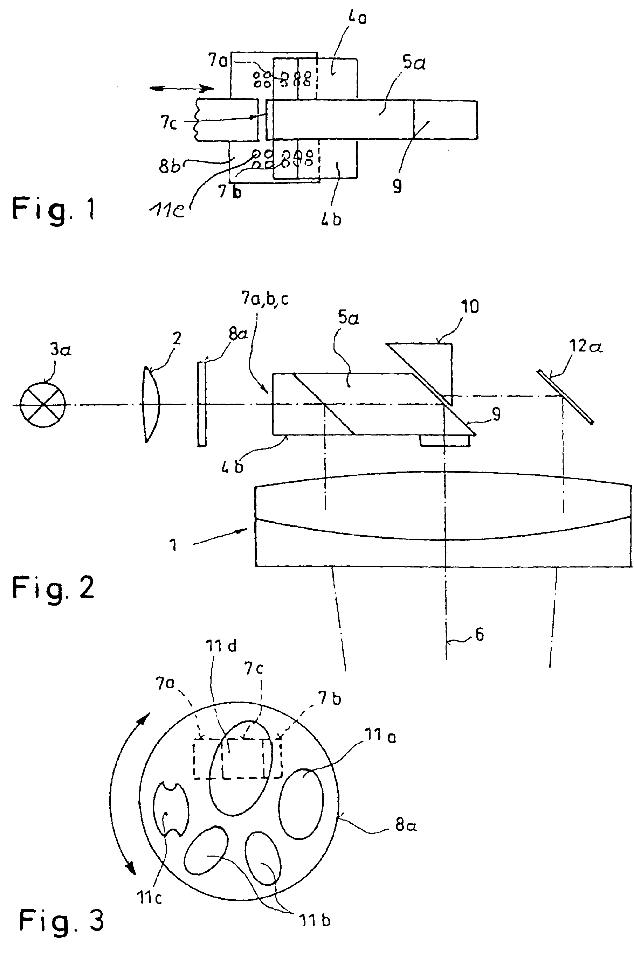

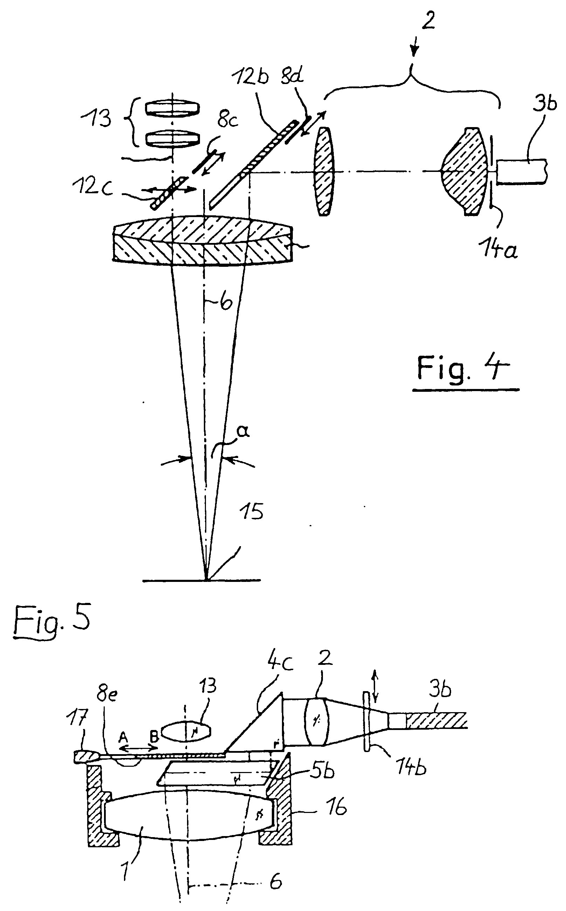

Fig.1 bis 3: Ein zweigeteiltes erstes Prisma 4a und 4b flankiert ein zweites Prisma 5a. Beispielsweise sind die Prismen miteinander verklebt, jedoch gegeneinander lichtdicht isoliert. Alle Prismen haben je eine Lichteintrittsfläche 7, die einer Beleuchtungsoptik 2 zugewandt ist. Beispielhaft ist eine Lichtquelle 3a dargestellt, die auch gut einen Lichtwellenleiter (3b gem Fig.4 und5) umfassen könnte. Die Zweiteilung des Prismas 4 ist nicht zwingend, jedoch bevorzugt, da sich dadurch eine gute Symmetrie bei der Ausleuchtung am Objekt ergibt. Die Anordnung könnte auch asymmetrisch sein.

Zwischen der Beleuchtungsoptik 2 und den Lichteintrittsflächen 7 ist optional eine verstellbare Blende 8a angeordnet. Diese kann beispielsweise so aussehen, wie in Fig.3 dargestellt. Dort erkennt man Fenster 11a-11d, die wahlweise vor die Lichteintrittsflächen 7 gebracht werden können. In Fig.3 ist das Fenster 11d vor die Lichteintrittsfläche 7c des zweiten Prismas gesetzt, sodass in diesem Fall nur die 0°-Beleuchtung mit voller Lichtstärke aktiviert ist. Die beiden ersten Prismenteile 4a und 4b erhalten praktisch keinen Lichtstrom. Würde man das Fenster 11d etwas weiterdrehen, so könnte dadurch eine Aufteilung des Lichtstromes zwischen der Lichteintrittsfläche 7c und einer von beiden Lichteintrittsflächen 7a oder 7b erfolgen.

Als krasse Alternative sind die Fenster 11b vorgesehen, die eine vollständige Abdunkelung der 0°-Beleuchtung bei gleichzeitiger Vollbeleuchtung des ersten Prismas 4a,b ermöglichen. Fenster 11a erlauben eine Vollbeleuchtung aller Prismen 4a,b und 5a und Fenster 11c erlaubt eine Vollbeleuchtung des Prismas 5a bei gleichzeitiger Reduktion der Beleuchtung des ersten Prismas 4a,b.

Die Prismen 4a, b könnten auch durchgängig einstückig ausgebildet sein, so dass die gesamte Eintrittsfläche 7a,b,c durch das Prisma 4 abgedeckt ist. Das Prisma 5a wäre dann als schmäleres Prisma an die Hypotenuse des Prismas 4 geklebt. Selbstverständlich können die Prismen 4a,b und 5a auch zusammen als einstückiges Prisma ausgebildet sein.

Unabhängig von der Blende 8a ist gegebenenfalls ein verschiebbares Prisma als Lichtbrechteil 10 vorgesehen, das mit einer planen Fläche an der Totalreflexionsfläche 9 des zweiten Prismas 5a verschiebbar ist. Wie nicht näher dargestellt, liegt es beispielsweise federbelastet an der Fläche 9 an. Durch die Unterbrechung der Totalreflexion wird ein Teil des Lichtstromes aus dem zweiten Prisma 5a nicht durch das Hauptobjektiv 1 gespiegelt, sondern durch den Lichtbrechteil 10 aus dem zweiten Prisma 5a abgeleitet. Unterstützend könnte hier ein Flüssigkeitsfilm, insbesondere ein Ölfilm auf der Kontaktfläche sein. Das könnte bei einer nichtgezeigten Variante eine Lichtvernichtung zur Folge haben, z.B. wenn die im Bild rechte Fläche des Lichtbrechteiles 10 schwarz gefärbt ist, oder wenn diese Fläche zwar durchsichtig ist, jedoch gegen eine schwarze Innentubusbeschichtung gerichtet ist.

Die Totalreflexion kann im Rahmen der Erfindung auch durch elektrooptische Schichten (z.B. LCD, Kristall- oder aufgedampfte Schichten) zwischen den beiden Prismen 4 und 5 gebrochen werden. Diese Schichten können - sofern sie elektronisch erregt, werden selektiv erregt werden, um derart die gewünschten Bereiche totalreflektierend bzw. reflexionsbrechend zu gestalten.

Beim vorliegenden Ausführungsbeispiel ist diese Fläche jedoch gegen eine weitere Spiegelfläche 12a gerichtet, die den abgezweigten Lichtstrom wiederum in einem kleinen Winkel oder parallel, je nach Spiegelstellung durch das Hauptobjektiv 1 gegen das Objekt lenkt. Bei Bedarf könnte die weitere Spiegelfläche noch verstellbar sein, um den Reflexionswinkel zu beeinflussen, wie jedoch nicht näher dargestellt ist.

Die vor die Lichtaustrittsflächen einschiebbare Blende 8b deckt nur einen Teil des Lichtstromes ab. Sie ist mit Löchern 11e versehen, die einen geringen Lichtdurchlass ermöglichen. Anstelle der Löcher können auch Streulinsen o.dgl. zur besseren Lichtverteilung angeordnet sein.

Anstelle der dargestellten Blende 8a oder der Löcherblende 8b können Filter, Gitter, bedampfte Glasplatten, Folien, o.dgl. treten.

In Fig.4 ist der Licht-Teil-und Lenkteil aus Spiegeln 12b und 12c gebildet. Ausserdem ist noch eine Optik 13 des mikroskopischen Strahlenganges angedeutet. Die Blenden 8c und 8d können unabhängig voneinander verstellt werden und sind wie die oben beschriebenen Varianten aufgebaut. Dieses Prinzip fällt auch dann unter die Erfindung, wenn nur ein Spiegel mit einer neuen erfindungsgemässen Blende 8d oder 8c abdeckbar ist. Die Beleuchtungsoptik 2 ist in Fig. symbolisch mit zwei Linsen dargestellt, denen eine Blende 14a vorgeschaltet ist. Solche Blenden sind an sich standardmässig bei vielen Geräten vorgesehen und dienen der Randbegrenzung und/oder der Abdunkelung des gesamten Beleuchtungslichtes. Eine selektive Abdunkelung ist dadurch nicht möglich. Eine vergleichbare Blende 14b ist in Fig.5 gezeigt. In Fig.5 ist die verschiebbare Blende 8e erfindungsgemäss ausgebildet und ermöglicht so ein selektives aber nicht vollständiges Abdunkeln der Beleuchtung unter kleinem Winkel. Die Blende 8e ist über einen händisch oder motorisch betätigbaren Schieber 17 bedienbar. Zum Haupobjektiv 1 ist noch eine Fassung 16 dargestellt.

## Patentansprüche

1. Beleuchtungseinrichtung an einem Operationsmikroskop mit einem Hauptobjektiv (1) an einer Hauptachse (6) und einer Beleuchtungsoptik (2) einer Lichtquelle (3), wobei zwischen dem Hauptobjektiv (1) und der Beleuchtungsoptik (2) eine Licht-Teil-und Lenkvorrichtung (12b,12c) vorgesehen ist, durch die im Betriebszustand die Umlenkung eines Teils des Lichtstromes der Beleuchtungsoptik (2) in einem kleinen Winkel zur Hauptachse (6) und ein anderer Teil des Lichtstromes der Beleuchtungsoptik (2) parallel zur Hauptachse (6) bewirkt wird und wobei ein Teil der Licht-Teil- und Lenkvorrichtung (4, 5, 12b, 12c) durch eine Blende abdeckbar ist, dadurch **gekennzeichnet, dass** die Blende nicht vollständig lichtundurchlässig ist.

2. Beleuchtungseinrichtung nach Anspruch 1, **dadurch gekennzeichnet,** dass die Blende als - vorzugsweise farbneutrales - Lichtfilter ausgebildet ist.

3. Beleuchtungseinrichtung nach Anspruch 1, **dadurch gekennzeichnet,** dass die Blende zwischen der Beleuchtungsoptik (2) und wenigstens einer Eintrittsfläche (7) der Licht-Teil- und Lenkvorrichtung (4, 5, 12b, 12c) eine angeordnet bzw. einschiebbar ist.

4. Beleuchtungseinrichtung nach Anspruch 3, **dadurch gekennzeichnet,** dass die Blende (8) eine an sich als lichtundurchlässige Scheibe mit selektiv vorschaltbaren Lichtdurchlässen (11) aufgebaut ist, die - dem Bedarf entsprechend - den Beleuchtungsoptikstrahlengang auf unterschiedliche Eintrittsflächen (7) in die Licht-Teil- und Lenkvorrichtung in unterschiedlichem Umfang freigeben.

5. Beleuchtungseinrichtung nach einem der vorhergehenden Ansprüche, **dadurch gekennzeichnet,** dass die Blende (8) als LCD aufgebaut ist, das - vorzugsweise elektronisch angesteuert - frei wählbare Anteile der Eintrittsflächen (7) freistellt.

6. Beleuchtungseinrichtung nach einem der vorhergehenden Ansprüche, **dadurch gekennzeichnet,** dass die Blende (8) als Lochblech oder Lochfolie ausgebildet ist.

7. Beleuchtungseinrichtung nach Anspruch 6, **dadurch gekennzeichnet,** dass die Blende als bedampfte Glas- oder Kunststoffplatte aufgebaut ist.

8. Beleuchtungseinrichtung nach Anspruch 7, **dadurch gekennzeichnet,** dass die Blende (8) als teildurchlässige Spiegelfläche ausgebildet ist, die, aus der Licht- Teil- und Lenkvorrichtung (10) austretende Strahlen, zum Teil wieder in Richtung zur Lichtquelle (3) umlenkt.

9. Beleuchtungseinrichtung nach einem der vorhergehenden Ansprüche, **dadurch gekennzeichnet,** dass die Blende (8) als Metall- oder Kunststoffgitter ausgebildet ist.

10. Beleuchtungseinrichtung nach einem der vorhergehenden Ansprüche, **dadurch gekennzeichnet,** dass vergleichbar der Blende (8) zusätzlich oder alternativ ein selektives Farbwahlfilter vorgesehen ist.

11. Beleuchtungseinrichtung nach einem der vorhergehenden Ansprüche, **dadurch gekennzeichnet,** dass die Blende- insbesondere an lichtdurchlässigen Stellen- optische Elemente und/oder Markierungen - beispielsweise Strichkreuze o.dgl. - zur Beeinflussung des sie durchdringenden Teil-Beleuchtungsstrahlenganges trägt.

12. Beleuchtungseinrichtung an einem Operationsmikroskop mit einem Hauptobjektiv (1) um eine Hauptachse (6) und einer Beleuchtungsoptik (2) einer Lichtquelle (3), **dadurch gekennzeichnet,** dass zwischen dem Hauptobjektiv (1) und der Beleuchtungsoptik (2) eine Licht-Teil- und Lenkvorrichtung (4,5) vorgesehen ist, von der im Betriebszustand die Umlenkung eines Teils des Lichtstromes der Beleuchtungsoptik (2) in einem kleinen Winkel zur Hauptachse (6) und die Umlenkung eines anderen Teils des Lichtstromes der Beleuchtungsoptik (2) parallel zur Hauptachse (6) bewirkt, wobei die Licht-Teil- und Lenkvorrichtung (4,5) so angeordnet ist, dass sie mit ihrer Lichteintrittsfläche (7) so der Beleuchtungsoptik (2) zugewandt sind, dass wenigstens ein Teillichtstrom durch die Licht-Teil-und Lenkvorrichtung (4,5) vom anderen Teillichtstrom unabhängig regelbar ist und dass für die Regelung eine Blende vorgesehen ist, die den betreffenden Lichtteilstrom nicht vollständig abdecken kann.
